# EUROPÄISCHE PATENTSCHRIFT

(11) **EP 0 949 233 B1**
(45) Veröffentlichungstag und Bekanntmachung des Hinweises auf die Patenterteilung: **02.07.2003**
(21) Anmeldenummer: 99106109.4
(22) Anmeldetag: 01.04.1999
(51) Int. Cl.: C07C 29/141, C07C 31/26, C07D 307/62, C07C 29/145

(54) **Verfahren zur Herstellung von Zuckeralkoholen**
Process for the preparation of sugar alcohols
Procédé pour la préparation d'alcools de sucre

(30) Priorität: 07.04.1998 DE 19815639
(43) Veröffentlichungstag der Anmeldung: 13.10.1999
(60) Teilanmeldung: 02008233.5
(73) Patentinhaber: BASF AKTIENGESELLSCHAFT, 67056 Ludwigshafen (DE)
(72) Erfinder: Brunner, Melanie, Dr., 67105 Schifferstadt (DE); Breitscheidel, Boris, Dr., 67117 Limburgerhof (DE); Henkelmann, Jochem, Dr., 68165 Mannheim (DE); Böttcher, Arnd, Dr., 67227 Frankenthal (DE)
(74) Vertreter: Isenbruck, Günter, Dr.

(56) Entgegenhaltungen:
- EP-A- 0 564 830
- EP-A- 0 803 488
- EP-A- 0 814 098
- US-A- 4 380 680
- US-A- 4 471 144
- DATABASE WPI Section Ch, Week 198232 Derwent Publications Ltd., London, GB; Class B05, AN 1982-66909E XP002123069 & JP 57 106630 A (TOA GOSEI CHEM IND LTD), 2. Juli 1982 (1982-07-02)

## Beschreibung

Die vorliegende Erfindung betrifft Verfahren zur Herstellung von Zuckeralkoholen durch Hydrierung von Zuckern unter Verwendung von Katalysatoren, die ein oder mehrere Metalle der VIII. Nebengruppe des Periodensystems als Aktivkomponente(n) umfassen.

Zuckeralkohole, wie z.B. Sorbitol, Mannitol, Maltitol und Xylitol finden breite Anwendung in der Lebensmittelindustrie, der Kosmetik, der Pharmazie und auf dem technischen Sektor.

Verfahren zur Herstellung von Zuckeralkoholen aus den korrespondierenden Zuckern durch Hydrierung, insbesondere diskontinuierliche Verfahren, bei denen pulverförmige Metall-Katalysatoren, wie z.B. Nickel-Katalysatoren, in einem Suspensionsverfahren zum Einsatz kommen, sind aus dem Stand der Technik bekannt (s. Ullmanns Encykl. der Technischen Chemie, Bd. 24, S. 772 (1983)).

Die EP-A 0 773 063 beschreibt ein kontinuierliches Verfahren zur Hydrierung von Zuckern an einem Raney-Ni-Al-Kontakt bei 130°C und 150 bar Druck.

Hydrierungen von Zuckern an geträgerten Ru-Kontakten sind ebenfalls beschrieben. Die US 4 471 144 beschreibt die Hydrierung von Kohlehydraten in wäßriger Lösung in Gegenwart eines Ruthenium auf θ-Al₂O₃-Katalysators. Die US 4 487 980 beschreibt ein ebensolches Verfahren in dem ein Katalysator mit einem Metall der VIII. Nebengruppe und TiO₂ als Träger eingesetzt wird. Die US 4 380 680 beschreibt die Verwendung eines Trägerkatalysators mit α-Al₂O₃ als Träger und einem Metall ausgewählt unter Os, Ru, Pd und Pt als Aktivkomponente bei der Hydrierung von Zuckern unter Erhalt von Zuckeralkoholen.

Eine Untersuchung bzgl. der bei derartigen Hydrierungen auftretenden Deaktivierung der verwendeten Katalysatoren am Beispiel der Hydrierung von Glucose unter Verwendung von Ru auf Al₂O₃ als Katalysator ist in einem wissenschaftlichen Artikel in Applied Catalysis A: General 87 (1992), S. 219-229 beschrieben.

Obwohl, wie sich aus obiger Zusammenfassung des Standes der Technik ergibt, bereits einige Verfahren zur Hydrierung von Zuckern bekannt sind, weisen die bislang verwendeten Katalysatoren nicht selten kurze Standzeiten auf, die durch eine Deaktivierung oder ein "Ausbluten" des Katalysators bedingt sind. Ferner kommt es während der Hydrierung häufig zu einer merklichen Epimerisierung, Zersetzung oder Polymerisation der Zuckeralkohole unter den gewählten Bedingungen.

Demgemäß besteht eine Aufgabe der vorliegenden Erfindung darin, neue Verfahren zur Hydrierung von Zuckern bereit zu stellen, in denen spezielle Katalysatoren mit einem oder mehreren Metallen der VIII. Nebengruppe des Periodensystems als Aktivmetall eingesetzt werden. Mit diesen neuen Verfahren soll es insbesondere ermöglicht werden, nahezu Epimeren-freie Zukkeralkohole in sehr hohen Ausbeuten bei nahezu vollständigem Umsatz zu erhalten. Darüberhinaus soll gegenüber den herkömmlichen Verfahren lediglich ein minimaler Anteil an Nebenprodukten bzw. Zersetzungsprodukten während der Hydrierung anfallen, um eine nachfolgende Aufarbeitung der Zuckeralkohole in einfacher und ökonomischer Weise durchführen zu können.

Demgemäß betrifft die vorliegende Erfindung ein Verfahren zur Hydrierung eines Zuckers oder eines Gemisches aus zwei oder mehr davon, das die folgende Stufe umfaßt:
Inkontaktbringen des Zuckers oder des Gemisches aus zwei oder mehr davon mit Wasserstoff in Gegenwart eines Katalysators, wobei ein Zuckeralkohol oder ein Gemisch aus zwei oder mehr davon erhalten wird, dadurch gekennzeichnet, daß der Katalysator als Aktivmetall mindestens ein Metall der VIII. Nebengruppe des Periodensystems alleine oder zusammen mit mindestens einem Metall der I. oder VII. Nebengruppe des Periodensystems in einer Menge von 0,01 bis 30 Gew.-%, bezogen auf das Gesamtgewicht des Katalysators, aufgebracht auf einem Träger, umfaßt, wobei 5 bis 50% des Porenvolumens des Trägers von Makroporen mit einem Porendurchmesser im Bereich von 50 nm bis 10.000 nm und 50 bis 95% des Porenvolumens des Trägers von Mesoporen mit einem Porendurchmesser im Bereich von 2 bis 50 nm gebildet werden, wobei sich die Summe der Porenvolumina zu 100% addiert.

Als Aktivmetall können prinzipiell alle Metalle der VIII. Nebengruppe des Periodensystems eingesetzt werden. Vorzugsweise werden als Aktivmetalle Platin, Rhodium, Palladium, Cobalt, Nickel oder Ruthenium oder ein Gemisch aus zwei oder mehr davon eingesetzt, wobei insbesondere Ruthenium als Aktivmetall verwendet wird. Unter den ebenfalls verwendbaren Metallen der I. oder VII. oder aber der I. und der VII. Nebengruppe des Periodensystems, die ebenfalls allesamt prinzipiell verwendbar sind, werden vorzugsweise Kupfer und/oder Rhenium eingesetzt.

Die Begriffe "Makroporen" und "Mesoporen" werden im Rahmen der vorliegenden Erfindung so verwendet, wie sie in Pure Appl. Chem., 45, S. 79 (1976) definiert sind, nämlich als Poren, deren Durchmesser oberhalb von 50 nm (Makroporen) oder deren Durchmesser zwischen 2 nm und 50 nm liegt (Mesoporen).

Der Gehalt des Aktivmetalls beträgt im allgemeinen 0,01 bis 30 Gew.-%, vorzugsweise 0,01 bis 5 Gew.-% und insbesondere 0,1 bis 5 Gew.-%, jeweils bezogen auf das Gesamtgewicht des verwendeten Katalysators, wobei die vorzugsweise verwendeten Gehalte nochmals bei der Diskussion des Katalysators angegeben sind.

Sofern zur Herstellung der erfindungsgemäßen Katalysatoren übliche Katalysatorträgersysteme, wie z.B. Aktivkohle, Siliciumcarbid, Aluminiumoxid, Siliciumdioxid, Titandioxid, Zirconiumdioxid, Magnesiumoxid, Zinkoxid oder deren Gemische, eingesetzt werden, werden diese jeweils in Kugel-, Strangoder Ringform, sofern sie als Festbettkontakt eingesetzt werden, sowie als Split, bzw. feinkörniges Granulat für die Verwendung in Suspension, verwendet. Weitere Details bzgl. dieser Trägersysteme sind der Diskussion beim Katalysator zu entnehmen.

Im Rahmen der vorliegenden Verfahren können prinzipiell alle Zucker eingesetzt werden. Dabei umfaßt der erfindungsgemäß verwendete Begriff "Zucker" sowohl Monosaccharide, wie z.B. Glucose, Mannose, Galactose, Talose, Fructose, Allose, Altrose, Idose, Gulose, Xylose, Ribose, Arabinose, Lyxsose, Threose und Erythrose, Di- und Trisaccharide wie z.B. Maltose, Lactose, Cellobiose, Sucrose, Melibiose und Raffinose, und Polysaccharide wie z.B. Stärke, Stärkezersetzungsprodukte, Cellulose und Cellulosezersetzungsprodukte, wie z.B. Dextrin, Glucosesirup, Cellulosehydrolysate und Stärkehydrolysate, wie z.B. Maisstärkehydrolysate.

Vorzugsweise werden im Rahmen der erfindungsgemäßen Verfahren Glucose zu Sorbitol, Mannose zu Mannitol, Fructose zu einer Mischung aus Sorbitol und Mannitol, Xylose zu Xylitol, Lactose zu Lactitol und Maltose zu Maltitol umgesetzt.

Die erfindungsgemäß verwendeten Katalysatoren enthalten ein oder mehrere Metalle der VIII. Nebengruppe des Periodensystems als Aktivkomponente(n) auf einem Träger, wie hierin definiert. Bevorzugt werden Ruthenium, Palladium und/oder Rhodium als Aktivkomponente(n) verwendet.

Die erfindungsgemäß verwendeten Katalysatoren können technisch hergestellt werden durch Auftragen mindestens eines Aktivmetalls der VIII. Nebengruppe des Periodensystems, vorzugsweise Ruthenium oder Palladium und gegebenenfalls mindestens einem Metall der I. oder VII. Nebengruppe des Periodensystems auf einem geeigneten Träger. Die Auftragung kann durch Tränken des Trägers in wäßrigen Metallsalzlösungen, wie z.B. Ruthenium- oder Palladiumsalzlösungen, durch Aufsprühen entsprechender Metallsalzlösungen auf den Träger oder durch andere geeignete Verfahren erreicht werden. Als Metallsalze zur Herstellung der Metallsalzlösungen eignen sich die Nitrate, Nitrosylnitrate, Halogenide, Carbonate, Carboxylate, Acetylacetonate, Chlorkomplexe, Nitritokomplexe oder Aminkomplexe der entsprechenden Metalle, wobei die Nitrate und Nitrosylnitrate bevorzugt sind.

Bei Katalysatoren, die mehrere Aktivmetalle auf den Träger aufgetragen enthalten, können die Metallsalze bzw. Metallsalzlösungen gleichzeitig oder nacheinander aufgebracht werden.

Die mit der Metallsalzlösung beschichteten bzw. getränkten Träger werden anschließend getrocknet, wobei Temperaturen zwischen 100°C und 150°C bevorzugt sind. Wahlweise können diese Träger bei Temperaturen zwischen 200°C und 600°C, vorzugsweise 350°C bis 450°C calciniert werden. Anschließend werden die beschichteten Träger durch Behandlung in einem Gasstrom, der freien Wasserstoff enthält, bei Temperaturen zwischen 30°C und 600°C, vorzugsweise zwischen 100°C und 450°C und insbesondere 100°C bis 300°C aktiviert. Der Gasstrom besteht vorzugsweise aus 50 bis 100 Vol.-% H₂ und 0 bis 50 Vol.-% N₂.

Werden auf die Träger mehrere Aktivmetalle aufgetragen und erfolgt das Auftragen nacheinander, so kann der Träger nach jedem Auftragen bzw. Tränken bei Temperaturen zwischen 100°C und 150°C getrocknet werden und wahlweise bei Temperaturen zwischen 200°C und 600°C calciniert werden. Dabei kann die Reihenfolge, in der die Metallsalzlösung aufgetragen oder aufgetränkt werden, beliebig gewählt werden.

Die Metallsalzlösung wird in einer solchen Menge auf den/die Träger aufgebracht, daß der Gehalt an Aktivmetall 0,01 bis 30 Gew.-%, vorzugsweise 0,01 bis 10 Gew.-%, weiter bevorzugt 0,01 bis 5 Gew.-%, und insbesondere 0,3 bis 1 Gew.-% bezogen auf das Gesamtgewicht des Katalysators, beträgt.

Die Metalloberfläche auf dem Katalysator beträgt insgesamt vorzugsweise 0,01 bis 10 m²/g, besonders bevorzugt 0,05 bis 5 m²/g und weiter bevorzugt 0,05 bis 3 m²/g des Katalysators. Die Metalloberfläche wurde durch das Chemisorptionsverfahren gemessen, wie es in J. LeMaitre et al., *"Characterization of Heterologous Catalysts*", Hrsg. Francis Delanney, Marcel Dekker, New York (1984), S. 310 - 324, beschrieben ist.

Im erfindungsgemäß verwendeten Katalysator beträgt das Verhältnis der Oberflächen des mindestens einen Aktivmetalls und des Katalysatorträgers weniger als 0,3, vorzugsweise weniger als 0,1 und insbesondere 0,05 oder weniger, wobei der untere Grenzwert bei 0,0005 liegt.

Die zur Herstellung der erfindungsgemäß verwendeten Katalysatoren verwendbaren Trägermaterialien besitzen Makroporen und Mesoporen.

Dabei weisen die erfindungsgemäß verwendbaren Träger eine Porenverteilung auf, der gemäß 5 bis 50%, vorzugsweise 10 bis 45%, weiter bevorzugt 10 bis 30 und insbesondere 15 bis 25% des Porenvolumens von Makroporen mit Porendurchmesser im Bereich von 50 nm bis 10.000 nm und 50 bis 95%, vorzugsweise 55 bis 90%, weiter bevorzugt 70 bis 90% und insbesondere 75 bis 95% des Porenvolumens von Mesoporen mit einem Porendurchmesser von 2 bis 50 nm gebildet werden, wobei sich jeweils die Summe der Porenvolumina zu 100% addiert.

Das Gesamtporenvolumen der erfindungsgemäß verwendeten Träger beträgt 0,05 bis 1,5 cm³/g, vorzugsweise 0,1 bis 1,2 cm³/g und insbesondere 0,3 bis 1,0 cm³/g. Der mittlere Porendurchmesser der erfindungsgemäß verwendeten Träger beträgt 5 bis 20 nm, vorzugsweise 8 bis 15 nm und insbesondere 9 bis 12 nm.

Vorzugsweise beträgt die Oberfläche des Trägers 50 bis 500 m²/g, weiter bevorzugt 200 bis 350 m²/g und insbesondere 250 bis 300 m²/g des Trägers.

Die Oberfläche des Trägers wird nach dem BET-Verfahren durch N₂-Adsorption, insbesondere nach DIN 66131, bestimmt. Die Bestimmung des mittleren Porendurchmesser und der Größenverteilung erfolgt durch Hg-Porosimetrie, insbesondere nach DIN 66133.

Obwohl prinzipiell alle bei der Katalysatorherstellung bekannten Trägermaterialien, d.h. die die oben definierte Porengrößenverteilung aufweisen, eingesetzt werden können, werden vorzugsweise Aktivkohle, Siliciumcarbid, Aluminiumoxid, Siliciumdioxid, Titandioxid, Zirkoniumdioxid, Magnesiumoxid, Zinkoxid oder deren Gemische, weiter bevorzugt Aluminiumoxid und Zirkoniumdioxid, eingesetzt.

Weitere Details bezüglich des Katalysators bzw. zu seiner Herstellung sind der DE-A 196 24 485 zu entnehmen.

### DIE VERFAHRENSFÜHRUNG

Im Rahmen der erfindungsgemäßen Verfahren wird die Hydrierung im allgemeinen bei einer Temperatur von 50 bis 140°C, vorzugsweise 80 bis 120°C durchgeführt. Die dabei verwendeten Drücke liegen in der Regel oberhalb von 50 bar, bevorzugt 80 bis 300 bar, besonders bevorzugt 100 bis 160 bar.

Die erfindungsgemäßen Verfahren können entweder kontinuierlich oder diskontinuierlich durchgeführt werden, wobei die kontinuierliche Verfahrensführung bevorzugt ist. Bei der kontinuierlichen Verfahrensführung können die jeweiligen Katalysatoren als Festbettkatalysatoren oder in Suspension eingesetzt werden. Vorzugsweise werden die erfindungsgemäßen Katalysatoren als Festbettkontakte eingesetzt. In Suspensionsfahrweise werden die erfindungsgemäßen Verfahren vorzugsweise in einem Reaktor durchgeführt, der eine Vorrichtung umfaßt, die Öffnungen oder Kanäle mit einem hydraulischen Durchmesser von 0,5 bis 20 mm, vorzugsweise 1 bis 10 mm und insbesondere 1 bis 3 mm aufweist, einer sogenannten Blasensäule, durchgeführt. Weitere Details bzgl. dieses speziellen Reaktors sind der DE-A 196 11 976 zu entnehmen.

Bei der kontinuierlichen Verfahrensführung beträgt die Menge des zur Hydrierung vorgesehenen Zuckers 0,05 bis 3 kg/l Katalysator pro Stunde, weiter bevorzugt 0,1 bis 1 kg/l Katalysator pro Stunde.

Als Hydriergase können beliebige Gase verwendet werden, die freien Wasserstoff enthalten und keine schädlichen Mengen an Katalysatorgiften, wie beispielsweise CO, aufweisen. Beispielsweise können Reformerabgase verwendet werden. Vorzugsweise wird reiner Wasserstoff als Hydriergas verwendet.

Die umzusetzenden Zucker werden in reiner Form als wäßrige Lösungen eingesetzt. Vorzugsweise betragen die Konzentrationen des Zuckers 15 bis 70 Gew.-%, weiter bevorzugt 30 bis 55 Gew.-%, bezogen auf das Gesamtgewicht der wäßrigen Lösung.

Der pH-Wert der wäßrigen Lösung beträgt im allgemeinen 3,5 bis 10, vorzugsweise 3,5 bis 8. Die wäßrigen Zuckerlösungen sind aufgrund geringfügiger Verunreinigungen durch Zuckersäuren im allgemeinen neutral oder schwach sauer. Sie können auf eine dem Fachmann bekannte Weise auf den gewünschten pH-Wert eingestellt werden. Prinzipiell kann die Hydrierung der Zucker auch ohne Veränderung des pH-Wertes durchgeführt werden, wobei eine spätere - ggf. aufwendige - Abtrennung der Substanzen, die zur pH-Wert-Einstellung eingesetzt werden, entfällt.

Im erfindungsgemäßen Verfahren ist der Umsatz der Zucker bei der Hydrierung nahezu vollständig. Es konnte maximal 0,1 Gew.-% des eingesetzten Zuckers in der Produktlösung nachgewiesen werden. Die Zuckeralkohole fallen in nahezu Epimeren-freier Form an, d.h. der Gehalt an Epimeren im Produkt beträgt im allgemeinen nicht mehr als 1 Gew.-%, vorzugsweise nicht mehr als 0,5 Gew.-%, und mit hoher Reinheit (> 99 %) an. Der Gehalt an Schwermetallen liegt im allgemeinen unter 10, vorzugsweise unter 5 und insbesondere unter 1 ppm.

Demgemäß betrifft die vorliegende Erfindung insbesondere die Verwendung der hierin beschriebenen Verfahren zur Herstellung von Zuckeralkoholen, die nahezu Epimeren-frei sind.

Die nach Abschluß der Hydrierung anfallenden, den/die gewünschten Zuckeralkohol(e) enthaltenden Produktlösungen können, falls dies erwünscht ist, nach üblichen Methoden, wie z.B. Sprühtrocknung, Gefriertrocknung, Trokkenwalzen oder Verdampfen, aufkonzentriert werden.

Jedenfalls können sie ohne weitere Reinigungsstufe konzentriert oder unkonzentriert weiter verarbeitet werden.

Die so erhaltenen Zuckeralkohole, insbesondere Sorbitol, können als Lebensmittelzusatzstoff, insbesondere als Süßstoffe, Feuchtigkeitsstabilisatoren in Nahrungsmitteln sowie in Kosmetika und pharmazeutischen Produkten, als Grundstoffe für die Polyurethan-Herstellung, insbesondere zur Herstellung von harten Polyurethan-Schäumen verwendet werden.

Das erfindungsgemäß hergestellte Sorbitol kann insbesondere zur Synthese von Vitamin C verwendet werden.

Somit betrifft die vorliegende Erfindung ferner ein Verfahren zur Synthese von Vitamin C ausgehend von Sorbitol, das dadurch gekennzeichnet ist, daß das Ausgangsmaterial Sorbitol gemäß einem der hierin beschriebenen Verfahren erhalten wird.

Das erfindungsgemäße Verfahren bzw. die Verwendung der hierin beschriebene Katalysatoren bei der Hydrierung von Zuckern zu Zuckeralkoholen bringt insbesondere dahingehend Vorteile, daß in den bei der Hydrierung erhaltenen Zuckeralkoholen keine nennenswerten Spuren von Metallen in kolloider oder ionischer Form, wie sie z.B. durch Zersetzung oder "Ausbluten" der Katalysatoren, insbesondere durch den chelatisierenden Effekt der Polyhydroxyverbindungen, entstehen können, nachzuweisen sind. Die Metallgehalte der Rohprodukte betragen im allgemein nicht mehr als 1 ppm. Somit entfällt die bei zahlreichen Verfahren des Standes der Technik notwendige Abtrennung der Schwermetalle aus den jeweiligen als Rohprodukt erhaltenen Zuckeralkoholen. Die Verfahren sind somit weitaus einfacher, kostengünstiger und ökologischer durchzuführen. Die als Rohprodukte anfallenden Zuckeralkohole erfüllen im allgemeinen die handelsüblichen Reinheitsbedingungen, etwa nach Deutschem Arzneibuch (DAB), Food Chemical Codex (FCC) oder Joint Experts Commitee on Food Additives (JECFA), und brauchen daher vor der Weiterverarbeitung z.B. im Lebensmittelbereich nicht weiter aufgereinigt zu werden.

Die vorliegende Erfindung soll nunmehr anhand eines Beispiels weiter erläutert werden.

### Beispiel

### Katalysatorherstellung

Ein meso-/makroporöser Aluminiumoxidträger in Form von 4 mm-Extrudaten, der eine BET-Oberfläche von 238 m²/g und ein Porenvolumen von 0,45 ml/g besaß, wurde mit einer wäßrigen Ruthenium-(III)-nitrat-Lösung, die eine Konzentration von 0,8 Gew.-% aufwies, getränkt. 0,15 ml/g (ungefähr 33% des Gesamtporenvolumens) der Poren des Trägers besaßen einen Durchmesser im Bereich von 50 nm bis 10.000 nm und 0,30 ml/g (ungefähr 67% des Gesamtporenvolumens) der Poren des Trägers wiesen einen Porendurchmesser im Bereich von 2 bis 50 nm auf. Das während des Tränkens vom Träger aufgenommene Lösungsvolumen entsprach dabei in etwa dem Porenvolumen des verwendeten Trägers.

Anschließend wurde der mit der Ruthenium-(III)-nitrat-Lösung getränkte Träger bei 120°C getrocknet und bei 200°C im Wasserstoffstrom aktiviert (reduziert). Der so hergestellte Katalysator enthielt 0,5 Gew.-% Ruthenium, bezogen auf das Gewicht des Katalysators.

### Hydrierung von Glucose

In einem 300 ml-Druckreaktor wurden 7g des Ru-Katalysators gemäß des Herstellungsbeispiels in einem Katalysator-Korbeinsatz vorgelegt und mit 150g (0,42 mol) einer 50 gew.-%igen Glucose-Lösung (pH-Wert 5,5) versetzt. Die Hydrierung wurde mit reinem Wasserstoff bei einem konstanten Druck von 150 bar und einer Temperatur von 100°C durchgeführt. Es wurde solange hydriert, bis kein Wasserstoff mehr aufgenommen wurde (10h). Der Reaktor wurde anschließend entspannt. Der Umsatz an Glucose betrug 99,95%. Die Ausbeute an Sorbitol lag bei 99,1%, die Ausbeute an Mannitol lag bei ca. 0,5%, jeweils bezogen auf die Gesamtmenge der eingesetzten Glucose. Der Ruthenim-Gehalt in den erhaltenen Zuckeralkoholen lag unter 1 ppm.

## Patentansprüche

1. Verfahren zur Hydrierung eines Zuckers oder eines Gemisches aus zwei oder mehr davon, das die folgende Stufe umfaßt:
Inkontaktbringen des Zuckers oder des Gemisches aus zwei oder mehr davon mit Wasserstoff in Gegenwart eines Katalysators, wobei ein Zuckeralkohol oder ein Gemisch aus zwei oder mehr davon erhalten wird, **dadurch gekennzeichnet, daß** der Katalysator als Aktivmetall mindestens ein Metall der VIII. Nebengruppe des Periodensystems alleine oder zusammen mit mindestens einem Metall der I. oder VII. Nebengruppe des Periodensystems in einer Menge von 0,01 bis 30 Gew.-%, bezogen auf das Gesamtgewicht des Katalysators, aufgebracht auf einem Träger, umfaßt, wobei 5 bis 50% des Porenvolumens des Trägers von Makroporen mit einem Porendurchmesser im Bereich von 50 nm bis 10.000 nm und 50 bis 95% des Porenvolumens des Trägers von Mesoporen mit einem Porendurchmesser im Bereich von 2 bis 50 nm gebildet werden, wobei sich die Summe der Porenvolumina zu 100% addiert.

2. Verfahren nach Ansprüch 1, wobei der Zucker ausgewählt wird aus der Gruppe bestehend aus Glucose, Mannose, Fructose, Xylose, Lactose, Maltose und Gemischen aus zwei oder mehr davon.

3. Verfahren nach Ansprüch 1 oder 2, **dadurch gekennzeichnet, daß** der Katalysator als Festbett vorliegt und das Verfahren kontinuierlich durchgeführt wird.

4. Verfahren zur Synthese von Vitamin C ausgehend von Sorbitol, das die folgende Stufe umfaßt:
Herstellung von Sorbitol ausgehend von Glucose mittels eines Verfahrens gemäß einem der Ansprüche 1 bis 3.

5. Verwendung eines Verfahrens gemäß einem der Ansprüche 1 bis 4 zur Herstellung von Zuckeralkoholen mit einem Epimeren-Gehalt von weniger als 1 Gew.-%.

## Claims

1. A process for the hydrogenation of a sugar or a mixture of two or more thereof, which comprises the following step:
Bringing the sugar or the mixture of two or more thereof into contact with hydrogen in the presence of a catalyst to give a sugar alcohol or a mixture of two or more thereof, wherein the catalyst comprises as active metal at least one metal of transition group VIII of the Periodic Table, either alone or together with at least one metal of transition group I or VII of the Periodic Table, in an amount of from 0.01 to 30% by weight, based on the total weight of the catalyst, applied to a support, where from 5 to 50% of the pore volume of the support is made up by macropores having a pore diameter in the range from 50 nm to 10,000 nm and from 50 to 95% of the pore volume of the support is made up by mesopores having a pore diameter in the range from 2 to 50 nm, where the sum of the pore volumes is 100%.

2. A process as claimed in claim 1, wherein the sugar is selected from the group consisting of glucose, mannose, fructose, xylose, lactose, maltose and mixtures of two or more thereof.

3. A process as claimed in claim 1 or 2, wherein the catalyst is present as a fixed bed and the process is carried out continuously.

4. A process for synthesizing vitamin C starting from sorbitol, which comprises the following step:
Preparing sorbitol from glucose by means of a process as claimed in any of claims 1 to 3.

5. The use of a process as claimed in any of claims 1 to 4 for preparing sugar alcohols having an epimer content of less than 1% by weight.

## Revendications

1. Procédé pour hydrogéner un sucre ou un mélange de deux ou plusieurs sucres, qui comprend les stades opératoires suivants :
mise en contact du sucre ou du mélange de deux ou plusieurs sucres avec l'hydrogène en présence d'un catalyseur, donnant un alcool de sucre ou un mélange de deux ou plusieurs alcools de sucre, ce procédé se caractérisant par le fait que le catalyseur contient en tant que métal actif au moins un métal du sous-groupe VIII de la Classification Périodique, seul ou avec au moins un métal du sous-groupe I ou VII de la Classification Périodique en quantité de 0,01 à 30 % du poids total du catalyseur, appliqué sur un support, de 5 à 50 % du volume de pores du support étant constitués de macropores à un diamètre de pore dans l'intervalle de 50 nm à 10 000 nm et 50 à 95 % du volume de pores du support constitués de mésopores à un diamètre de pore dans l'intervalle de 2 à 50 nm, la somme de ces volumes de pores représentant 100 %.

2. Procédé selon la revendication 1, selon lequel le sucre est choisi dans le groupe consistant en le glucose, le mannose, le fructose, le xylose, le lactose, le maltose et les mélanges de deux ou plusieurs d'entre eux.

3. Procédé selon la revendication 1 ou 2, **caractérisé par le fait que** le catalyseur est à l'état de lit fixe et **par le fait que** le procédé est mis en oeuvre en continu.

4. Procédé pour la synthèse de la vitamine C au départ du sorbitol, comprenant le stade opératoire suivant :
préparation du sorbitol au départ du glucose par un procédé selon l'une des revendications 1 à 3.

5. Utilisation d'un procédé selon l'une des revendications 1 à 4 pour la préparation d'alcools des sucres à une teneur en épimère inférieure à 1 % en poids.
